# EUROPEAN PATENT SPECIFICATION

(11) **EP 3 349 830 B1**
(45) Date of publication and mention of the grant of the patent: **26.11.2025**
(21) Application number: 16766035.6
(22) Date of filing: 06.09.2016
(51) Int. Cl.: A61M 15/00, A24F 47/00, A61M 15/06, A24F 40/10, A61M 11/04, A24F 40/485

(54) **AEROSOL PROVISION SYSTEM WITH VARIABLE AIRFLOW**
AEROSOLBEREITSTELLUNGSSYSTEM MIT VARIABLEM LUFTSTROM
SYSTÈME DE FOURNITURE D'AÉROSOL AVEC FLUX D'AIR VARIABLE

(30) Priority: 16.09.2015 GB 201516439
(43) Date of publication of application: 25.07.2018
(73) Proprietor: Nicoventures Trading Limited, London WC2R 3LA (GB)
(72) Inventor: HEPWORTH, Richard, London WC2R 3LA (GB); LEADLEY, David, London WC2R 3LA (GB); TRAN, My-Linh, London WC2R 3LA (GB)
(74) Representative: D Young & Co LLP
(86) International application number: PCT/GB2016/052738
(87) International publication number: WO 2017/046566

(56) References cited:
- WO-A1-2014/201671
- GB-A- 2 412 876
- US-A1- 2013 192 615

## Description

### Technical Field

The present invention relates to an aerosol provision system with an airflow adjuster for varying airflow through the system.

### Background

Aerosol provision systems such as e-cigarettes generally contain a reservoir of a source liquid containing a formulation, typically including nicotine, from which an aerosol is generated, such as through vaporisation or other means. Thus an aerosol source for an aerosol provision system may comprise a heating element coupled to a portion of the source liquid from the reservoir. When the heating element is activated it causes vaporisation of a small amount of the source liquid, which is thus converted to an aerosol for inhalation by the user. More particularly, such devices are usually provided with one or more air inlet holes located away from a mouthpiece of the system. When a user sucks on the mouthpiece, also known in the industry as a "drip tip", air is drawn through the inlet holes and past the aerosol source. There is an air flow path connecting the inlet holes to the aerosol source and on to an opening in the mouthpiece so that air drawn past the aerosol source continues along the flow path to the mouthpiece opening, carrying some of the aerosol from the aerosol source with it. The aerosol-carrying air exits the aerosol provision system through the mouthpiece opening for inhalation by the user.

Some aerosol provision systems are configured in two sections. An aerosol delivery section houses the reservoir of source liquid and one or more heating elements, and has the airflow path defined therethrough from the inlet hole(s) to the mouthpiece. A battery section houses a battery (which may be replaceable or rechargable) for providing electrical power to the heating element. An electrical connection is provided between the two sections. The sections can be separable from one another, in which case there is also a reversible mechanical connection between the sections.

The two sections can be arranged linearly so that the battery section is connected at the opposite end of the aerosol provision section to the mouthpiece. This gives a generally elongate device in which the battery section is aligned substantially along the direction of airflow in the flow path, and when the mouthpiece points upwards, as it does in use, the battery section is underneath the aerosol provision section.

The air inlet(s) can be located in a side wall of the aerosol delivery section just below the base of the reservoir, that is, the part of the reservoir remote from the mouthpiece. Some systems allow the user to vary the amount of air that can flow along the air flow path, to give some control over the amount of formulation that can be consumed per inhalation. Often, this variability is provided by an external adjustable element that partially covers the air inlet to enable the effective size of the inlet to be altered.

International Patent Application WO 2014/201671 A1 discloses a vent hole-adjustable electronic cigarette comprising a first smoke volume adjustment element and a second smoke volume adjustment element that are coaxially arranged with the outer tube. The first and second smoke volume adjustment elements constitute a smoke volume adjustment mechanism. The first and second smoke volume adjustment elements may respectively be a first suction nozzle cover and a second nozzle cover that are arranged at one end of the outer tube. Alternatively, the first and second smoke volume adjustment elements may respectively be a first lamp cap and a second lamp cap that are arranged at one end of the outer tube. Alternatively, the first and second smoke volume adjustment elements may be arranged at positions where the atomizer tube and the battery tube are connected to each other.

### Summary

The invention is defined in independent claim 1. Preferred embodiments are defined in the dependent claims. Any other embodiments not falling under the scope of the claims (or methods) are merely exemplary.

According to a first aspect of certain embodiments described herein, there is provided an aerosol delivery component for a vapour provision system, comprising: an air inlet; an airflow path connected to the air inlet and extending through the aerosol delivery component; a heating element in the airflow path for vaporising a source liquid delivered from a tank to form an aerosol; and an airflow adjuster for varying a level of airflow along the airflow path, the airflow adjuster allowing a user to alter an amount of air able to flow along the airflow path, and located in the airflow path downstream from the air inlet and upstream of the heating element, wherein the aerosol delivery component is mechanically and electrically connectable to a battery section of the vapour provision system, the battery section housing a battery connectable to the aerosol delivery component to provide electrical power to the heating element in the aerosol delivery component and said battery section having one or more buttons or switches operable by the user to deliver electrical power to one or more components in the aerosol delivery component.

The airflow adjuster may be spaced from the air inlet by a portion of the airflow path.

The airflow adjuster may comprise an element movable into and out of the airflow path to alter a size of a bore of the airflow path at the location of the element. The element may be movable by rotation or sliding.

In some embodiments, the aerosol delivery component comprises a first section having a first portion of the airflow path defined therein and a second section having a second portion of the airflow path defined therein, and the airflow adjuster comprises a planar element interposed between the first section and the second section and having at least one aperture through the planar element, the planar element being rotatable such that the aperture can be brought into and out of alignment with the airflow path.

The aerosol delivery component may further comprise a rotation limiter configured to prevent rotation of the planar element into a position in which there is no alignment of the aperture with the airflow path.

The at least one aperture may comprise at least two apertures circumferentially spaced around the planar element and spaced apart by a circumferential distance which is less than a circumferential dimension of the airflow path, so that there is at least partial alignment of an aperture and the airflow path for all rotational positions of the planar element.

One or both of the at least one aperture and the airflow path at the location of the planar element may have a dimension in a radial direction with respect to the axis of rotation of the planar element that is not constant over the circumferential extent of the aperture or path. For example, the said dimension in the radial direction may increase over the circumferential extent.

The airflow path may comprise two or more airflow paths at the location of the adjuster.

According to a second aspect of certain embodiments described herein, there is provided a vapour provision system comprising an aerosol delivery component according the first aspect, and a battery section for housing a battery connectable to the aerosol delivery component to provide electrical power to the heating element in the aerosol delivery component.

These and further aspects of certain embodiments are set out in the appended independent and dependent claims. It will be appreciated that features of the dependent claims may be combined with each other and features of the independent claims in combinations other than those explicitly set out in the claims. Furthermore, the approach described herein is not restricted to specific embodiments such as set out below, but includes and contemplates any appropriate combinations of features presented herein.

### Brief Description of the Drawings

Various embodiments will now be described in detail by way of example only with reference to the accompanying drawings in which:
Figure 1 shows a schematic side view of an aerosol provision system having an airflow adjuster in accordance with embodiments of the invention;
Figure 2 shows an exploded perspective partial view of part of an aerosol provision system having an airflow adjuster in accordance with some embodiments;
Figure 3 shows a plan view of an example airflow adjuster according to some embodiments;
Figure 4 shows a plan view of another example airflow adjuster according to some embodiments;
Figure 5 shows a plan view of a further example airflow adjuster according to some embodiments;
Figure 6 shows a schematic side view of sections of an aerosol provision system connected in accordance with some embodiments;
Figure 7 shows a schematic partial side view of sections of an aerosol provision system connected in accordance with other embodiments;
Figure 8 shows a schematic side view of a further aerosol provision system having an airflow adjuster in accordance with embodiments of the invention;
Figure 9 shows a plan view of a yet further example airflow adjuster according to some embodiments;
Figure 10 shows a plan view of a still further example airflow adjuster according to some embodiments; and
Figure 11 shows a plan view of still another example airflow adjuster according to some embodiments.

### Detailed Description

Aspects and features of certain examples and embodiments are discussed/ described herein. Some aspects and features of certain examples and embodiments may be implemented conventionally and these are not discussed/described in detail in the interests of brevity. It will thus be appreciated that aspects and features of apparatus and methods discussed herein which are not described in detail may be implemented in accordance with any conventional techniques for implementing such aspects and features.

As described above, the present disclosure relates to aerosol provision systems, such as e-cigarettes. Throughout the following description the term "e-cigarette" may sometimes be used; however, it will be appreciated this term may be used interchangeably with aerosol (vapour) provision system.

Directional terms such an "upper" and "lower" used in this description are used for the sake of convenience and brevity and for relevance to directions used in the Figures, and should not be considered limiting, since e-cigarettes may be held in any orientation.

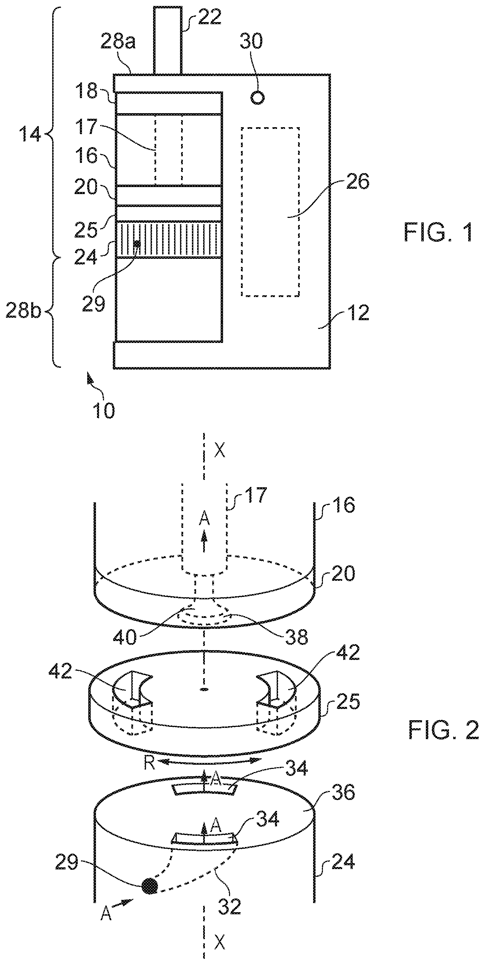

Figure 1 is a schematic diagram of an example aerosol/vapour provision system such as an e-cigarette 10 to which some embodiments are applicable. In this example, the e-cigarette 10 is a modular device with a "side-by-side" configuration, comprising a battery section or component 12 and an aerosol delivery section or component 14 which are mechanically and electrically connected together. The battery section 12 houses a battery 26 and has one or more buttons or switches 30 operable by a user to deliver electrical power to one or more components in the aerosol delivery section 14. The battery section 12, in this example, has two supporting sections 28a, 28b which extend laterally to receive and support the aerosol delivery section 14 in a position next to the battery section 12. In particular, a lower supporting section 28b is a connecting portion which makes the necessary electrical connections to the battery 26, and also provides a mechanical connection which may be, for example, a screw thread connection between cooperating screw threads on the connection portion 28b and the base of the aerosol delivery section 14. The aerosol delivery section 14 is cylindrical in this example, and at its base has a grip portion 24 provided with vertical grooves or other surface features to facilitate grip, so that a user can hold this part and rotate the aerosol delivery section 14 to disengage the screw threads. Once the threads are separated, the aerosol delivery section 14 can be lifted free from the battery section 12. The two sections may be provided to a consumer as a combined unit, or as separate items to be connected by the user, thereby allowing some customisation, and/or to allow either section to be replaced or refilled in the event of expiration of the battery 26 or emptying of the tank 16.

The aerosol delivery section 14 comprises the aforementioned grip section 24 at its base, above which is a tank base 20. A tank or reservoir 16 extends upwardly from this base 20 and is formed by transparent walls defining a cylindrical shape through which a user can conveniently observe liquid solution held in the tank. The walls need not be transparent, however. A tube or pipe 17 runs up through the tank 16, disposed along the central axis of the aerosol delivery section 14 in this example; this defines part of an airflow path that runs through the aerosol delivery section 14. Disposed within the pipe 17 are one or more wicks mounted within or around one or more heating coils (not shown). The wicks absorb liquid from the tank, the heating coils are heated when electrical current is supplied to them from the battery 26, and the liquid in the wicks is vaporised and carried away on air flowing through the pipe 17. A lid 18 is provided to close the upper end of the tank 16. The lid 18 can be removed to allow the tank 16 to be refilled when the aerosol delivery section 14 is disconnected from the battery section 12. The airflow path passes through the lid 18 to a mouthpiece 22 (which may or may not be removable) through which a user can inhale to generate the required airflow along the airflow path.

The opposite end of the air flow path within the aerosol delivery section 14 to the mouthpiece 22 is defined by at least one air inlet 29 in the outer surface of the aerosol delivery section 14 which connects to the pipe 17 via one or more channels or cavities. When a user inhales through the mouthpiece 22, air is taken in through the air inlet 29 to flow along the airflow path.

The system 10 also comprises an airflow adjuster 25, which in this example is positioned between the grip portion 24 and the tank base 20. The adjuster is an element that allows a user to alter the amount of air able to flow along the airflow path, and hence the amount of vaporised solution that can be inhaled in each inhalation.

Embodiments of the present invention propose that the air inlet be positioned downstream from the adjuster. For example, in Figure 1, the air inlet 29 is formed as a hole in the side wall of the aerosol provision section, in the grip portion 24. It might be positioned in a different section, for example an additional part positioned between the grip portion 29 and the adjuster 25 (not shown) or elsewhere on the vapour provision device. More than one hole might be used as the air inlet, spaced apart around the aerosol provision section or in the longitudinal direction; this reduces the risk that the user accidentally blocks the air inlet while holding the system to inhale. Regardless of the number of holes and the precise disposition, the air inlet 29 connects with one or more channels or cavities within the aerosol provision section 14 which are in air flow communication with the pipe 17, to form that part of the airflow path which is downstream from the heating element.

In accordance with embodiments, the airflow adjuster is positioned downstream from the air inlet, with respect to the direction of airflow along the airflow path during a user inhalation. This is in contrast to arrangements in which the amount of airflow may be adjusted by a movable element that covers the air inlet from the outside of the system, whereby the air inlet is downstream from the adjuster. Configurations according to the invention give more design freedom in the positioning of the air inlet (so that it can be located in a position less likely to become accidentally blocked, for example) while allowing the airflow adjuster to be retained in a conveniently accessible location readily apparent to the user. While Figure 1 shows the adjuster between the grip section and the tank base, it may be located in other positions along the airflow path which are downstream of the air inlet, in particular in components and systems including additional parts beyond those shown in Figure 1. In this example, note that the adjuster is upstream from the wick and the heating element.

Figure 2 shows a perspective exploded view of part of an aerosol provision section according to an embodiment. A cylindrical lower portion, such as the grip portion 24 from Figure 1, has an air inlet hole 29 in its side. This leads to a hollow channel 32 defined through the material of the lower portion 24 (which might be metal or plastic, for example). The channel 32 (which might also be a much larger cavity, its shape being unimportant) terminates in a first lower opening 34 in the upper face 36 of the lower portion 24. A second similar lower opening 34 is positioned diametrically opposite to the first aperture, in the upper face 36. This can be connected to a second air inlet (not shown) via a second channel (not shown) similar to the channel 32. Alternatively, the channel 32 may be shaped so as divide or fork or may be a single large cavity, so as to connect a single inlet 29 with both lower openings 34. Alternatively still, a single inlet 29, channel 32 and lower opening 34 may be provided. Each of the lower openings 34 is disposed radially outwardly from the centre of the upper face 36. In this example, each opening 34 has a curved arcuate shape, curving along a line substantially parallel to the circumferential edge of the upper face 36. In other examples, a larger number of inlets and lower openings might be used, connected by any configuration of channels or cavities. A plurality of inlets might connect to one opening.

A tank 16 has a base 20 as before, and a central tube 17 to carry air to the wick and heating element (not shown). Formed in the lower surface of the base 20 are a pair of upper openings 38 (one shown only). Channels 40 (one shown only) are defined in the material of the base 20 to connect the upper openings 38 to the pipe 17. If the lower portion has a single lower opening only, only one upper opening 38 need be provided. In either case, the upper openings 38 have a similar shape, size and location to the two lower openings 34 in the upper face 36 of the lower portion 24 in this example. Hence, the lower openings 34 and the upper openings 38 are aligned with one another, so that if the lower portion 24 and the tank 16 were placed in contact, the lower and upper openings would connect the channels 32 and 40 to form a continuous airflow path from the inlet 29 to the tube 17. Each pair of an upper opening and a lower opening and their corresponding channels can be considered as defining an airflow path, where all of these individual paths together collectively form the airflow path through the aerosol provision section. Having more than one path at this location, where the lower portion 24 meets the tank 16, provides for more flexibility in control of the airflow. Inhalation by a user through a mouthpiece connected to the tube 17 (see Figure 1) causes air to be drawn in through the air inlet 29, whence it flows along the channel 34 out of the lower aperture 34, into the upper aperture 38, through the channel 40 and into the tube 17, as shown by the arrows A. These elements together form an airflow path. Hence, the lower portion 24 has defined therein a first, lower, or downstream portion of the airflow path, and the tank base 20 has defined therein a second, upper, or upstream portion of the airflow path (which then extends along the tube 17 to the wick, heating element and eventually the mouthpiece)

However, disposed between the lower portion 24 and the tank base 20 is an airflow adjuster 25. In this example, the adjuster 25 is a planar element in the form of a plate or disc, formed from metal or plastic, for example. It has a diameter substantially the same as those of the cylindrical lower portion 24 and the tank base 20 and the tank 16, so that all these components can be stacked together to form a longer cylindrical component. The cylindrical shape facilitates attachment of the aerosol provision section 14 to the supporting section 28b of the battery section 12 by a screw thread, but it is not essential.

The airflow adjuster 25 has a pair of apertures 42 formed through its thickness. In this example, these have the same shape and size as the lower openings 34 and the upper openings 38. They are located at the same distance from the centre of the adjuster disc 25 as the lower openings are from the centre of the lower portion 24 and the upper openings are from the centre of the tank base 20. When the components are stacked together (i.e. brought into contact with one another compared to the spaced-apart exploded view of Figure 2) they are aligned along a central longitudinal axis X. They are mechanically coupled together such that a proper alignment of the lower portion 24 and the tank 16 is maintained for correspondence of the upper openings 38 with the lower openings 34, while the adjuster 25 is able to be rotated about the central axis X, indicated by the arrow R. This rotation can be employed to alter the position of the apertures 42 with respect the upper and lower openings 38, 34, such that the amount of overlap between the apertures and the openings changes. When the apertures 42 are aligned fully with the openings 34, 38 the airflow path is uninterrupted and unrestricted, and a maximum amount of airflow along the path is enabled. Rotation of the adjuster 25 from this position moves the apertures into a reduced alignment with the openings to reduce the amount by which the apertures 42 overlap with openings 34, 38. The openings are thereby partly blocked, and the amount of airflow possible along the airflow path is reduced.

A user can thereby rotate the adjuster 25 to alter the level of airflow as desired.

As depicted in Figure 2, the apertures 42 are wholly unaligned with the upper and lower openings 34, 38, so that the airflow path is completely blocked. Rotation through 90 degrees will achieve maximum alignment. It may be desired that complete blockage of the airflow path be unachievable, such as for safety reasons, so the system may be configured to ensure that the airflow path is always at least partly open. In an arrangement such as that of Figure 2 (where the openings and apertures are of limited circumferential extent) this might be achieved by limiting the amount of rotation of the adjuster 25 to be within a range where an overlap between the openings and the apertures is always maintained.

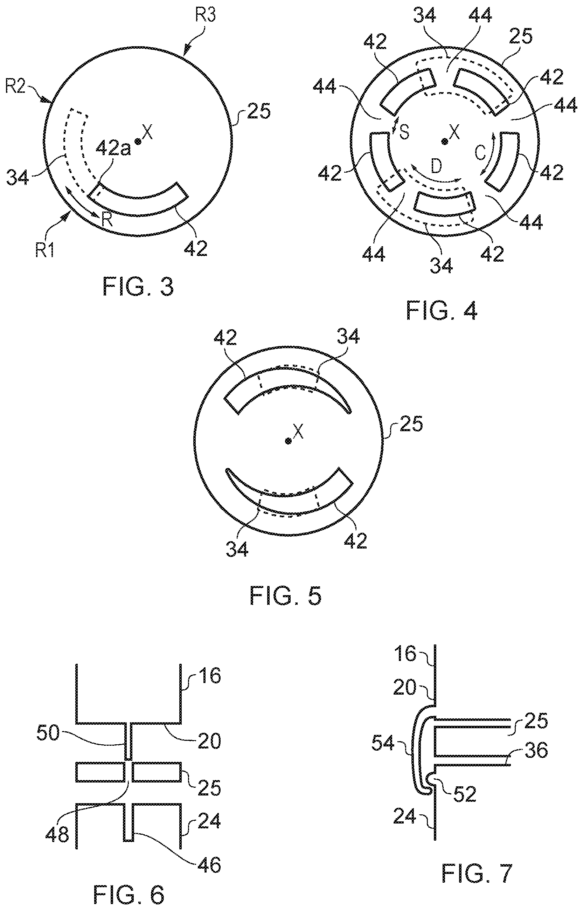

Figure 3 shows a plan view of the adjuster 25 with an arcuate aperture 42, and the position of the lower opening 34 underneath shown in phantom. For simplicity, this example has only one aperture 42 and one opening 34. If rotation of the adjuster 25 about the central axis X, as shown by the arrow R, is limited so that the end 42a of the aperture 42 can move from position R1 (minimum overlap and minimum airflow) only as far as position R2 (maximum overlap and maximum airflow), the airflow path will never be blocked. Further rotation to the position R3 might be permitted, so that the airflow can be adjusted from minimum to maximum and back to minimum by rotation in one direction only. The rotation can be limited in any way, as preferred, such as by the use of abutting elements, or a screw thread of limited extent. For example, a peg or post might protrude from an edge or end of the aperture 42, and extend into the opening 34. Rotation cannot then be extended past the positions at which the peg abuts either end of the opening 34.

An alternative approach to ensuring that the airflow path remains open is an appropriate configuration of the apertures and openings. In the Figure 1 arrangement there are many positions of the adjuster 25 in which there is no overlap between the apertures 42 and the openings 34, 38, so that the airflow path is blocked. A different arrangement can ensure that there is no position in which the path can become blocked. For example, the number of apertures and/or openings can be increased, or they may be made larger.

Figure 4 shows an example of such a configuration. The adjuster 25 is shown in plan view, and includes a plurality of apertures 42 disposed around the disc. Each aperture 42 has a circumferential extent C, and is spaced from the adjacent apertures by a circumferential distance S. The openings 34 (two in this example) in the underlying lower portion 24 are shown in phantom. They have a circumferential extent D which is longer than the spacing S. By arranging the spacings (which can partially block the openings 34) to be shorter than the length of the openings 34, it is not possible for there to be no overlap between at least one the aperture 42 and each opening 34, so the airflow path cannot be completely blocked. The lengths C, D and S and the number of apertures 42 can be chosen to give a desired level of adjustability.

As is clear from the foregoing, embodiments of the invention are not limited to any particular number of openings or apertures, provided there is at least one of each. Also, the relative sizes of the openings and apertures can be modified as required; they need not be the same as in Figures 1 and 2. The maximum bore of the airflow path at the point of adjustment (and hence the amount of achievable airflow) will be determined by the size defined by the edges of the openings and the apertures when the overlap between them is at a maximum. The bore is the width or size or area of the airflow path in the transverse or cross-sectional direction, that is, orthogonal to the airflow direction.

The examples so far have shown openings and apertures which have a size in the radial direction (from the centre to the circumference of the component in which they are defined) which is substantially constant. This is not essential; either the apertures, the openings or both may be otherwise shaped to achieve particular effects of flow control.

Figure 5 shows an example of such an embodiment, as a plan view of the adjuster 25. The openings 34 in the underlying lower portion 24 are show in phantom and have a constant radial dimension. In contrast, the apertures 42, in this example a pair oppositely disposed across the disc, are each shaped so as to have a dimension in the radial direction which varies along the circumferential length of the apertures, so that the apertures are tapered, being wider at one end and narrower at the other end (having an arcuate or curved triangular shape). Compared with an aperture of constant width, an increasing or decreasing width allows a greater change in the overlap between the aperture and the opening for the same amount of rotation. Thus, the adjustment is made more sensitive, or finer, and less external movement of the adjuster may be needed to achieve a particular range of adjustment. An equivalent effect is achieved if the shapes of the apertures 42 and openings 34 are reversed, or if both are made tapering but in opposite directions. Other shapes of either the aperture(s) or the opening(s) are also possible, and can be selected, together with the sizes, according to the maximum and minimum airflow desired and the amount of movement of the adjuster considered acceptable to achieve this range.

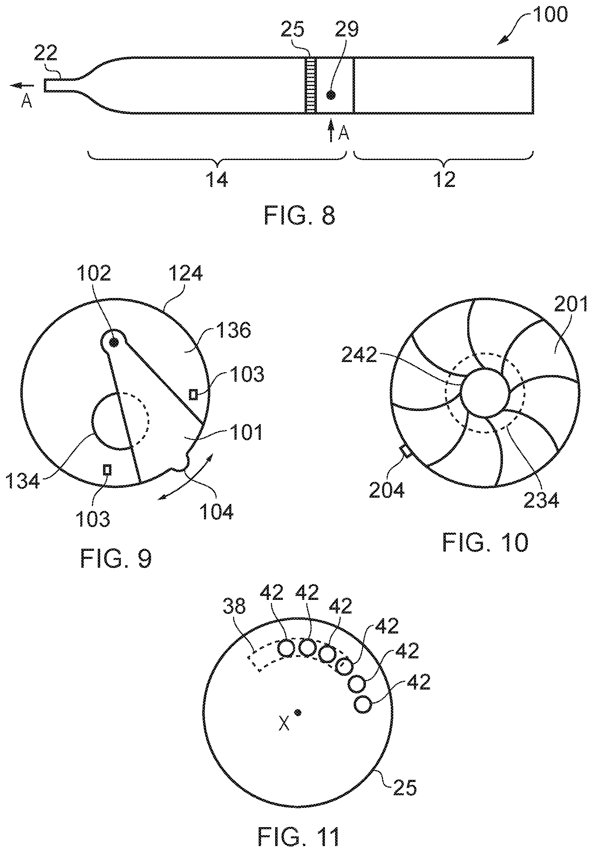

Figure 11 shows another example with an alternative arrangement of apertures. In this example, again shown as a plan view of the adjuster 25, a plurality of small apertures 42 are formed through the adjuster 25. The apertures 42 are spaced apart in the circumferential direction by a distance much less than the circumferential extent of the opening 38, and extend over a total circumferential distance roughly the same as the circumferential extent of the opening 38. By "small", it is meant that the size of the apertures 42 in the circumferential direction is very much less than that of the opening 38 in that direction. Rotation of the adjuster 25 about the axis X will bring a greater or lesser number of the small apertures 42 into alignment with the opening 38, thereby increasing or decreasing the airflow. In this case the effective bore of the airflow path is the area of all the small apertures 42 that are aligned with the opening 38. This configuration might be preferred if manufacturing a series of small holes is more readily accomplished that manufacturing one large aperture.

In these embodiments, rotational movement of the adjuster enables the adjustment. An appropriate mechanical connection between the components is therefore provided.

Figure 6 shows an exploded side view of part of an aerosol provision system with a first example of a mechanical connection for rotational movement. The base 20 of the tank 16 has a central spindle 50 protruding downwardly from its underside (the surface facing the adjuster 25). The adjuster 25 has a central hole 48. The lower portion 24 has a central socket 46. When assembled, the spindle 50 passes through the hole 48 and engages into the socket 46 (by a screw thread, a friction fit, gluing, welding, or any other attachment technique). The tank 16 and the lower portion 24 are thus secured together, with their openings in alignment to form the airflow path, and the adjuster is free to rotate about the spindle. The spindle might alternatively extend to an accessible underside of the lower portion 24 and be secured in the socket 46 by a nut, rivet or similar.

Figure 7 shows an enlarged external view of a side part of an aerosol provision system with a second example of a mechanical connection for rotation movement. The lower portion 24 has a lug protruding from its outer surface close to the upper face 36. The tank 16 has a clip 54 extending downwardly from its outer surface in the region of the base 20. When the tank 16 and the lower portion 24 are brought together with the adjuster disc 25 sandwiched between them, an end of the clip 54 which is hooked or recessed engages over the lug 52 to fasten the tank 16 and the lower portion 24 together. A moulded plastic clip can have flexibility and resilience to deform over the lug and then spring into position; metal clips may be formed to have the same action. Two or more pairs of lugs 52 and clips 54 may be provided around the aerosol provision section to achieve the required connection. The lugs 54 might be replaced by a collar extending around the lower portion 24, or by a circumferential recess or individual recesses in the wall of the lower portion 24 if the clip ends are sufficiently inwardly shaped. Individual lugs or recesses may facilitate alignment of the openings in the lower portion 24 and the tank base 20.

In the examples of Figures 6 and 7, the arrangements may be reversed with respect to those fastening parts which appear on the tank portion and those which appear on the lower portion. Other mechanical fastenings which allow rotation of the adjuster are also possible.

The adjuster 25 may have a ridged or otherwise textured outer surface (the part contiguous with the cylindrical outer walls of the tank base and the lower portion) to facilitate gripping by the user to make an adjustment to the airflow. The adjuster 25 may have a larger diameter than the tank base or the lower portion so that it protrudes slightly from the adjacent outer surface of the aerosol delivery section 14, again to facilitate gripping by the user. Instead of being circular, the adjuster may be shaped so that its outer surface is multifaceted (so that in plan view the adjuster is a polygon rather than a disc). This shaping also facilitates gripping. The adjuster may have markings on its outer surface to provide a visual indication of the required movement for airflow adjustment and the direction required for increasing and decreasing airflow. These markers may correspond with markings on one of the adjacent fixed parts, namely the lower portion or the tank base, so that the amount of rotation made or required from a defined position is indicated.

In the examples above, the apertures 42 are substantially straight through-holes extending through the thickness of the adjuster 25 and orthogonal to the surface, and the tank base 20 and the lower portion 24 are positioned with respect to one another so that their openings are aligned. This is not essential, however. If the thickness of the adjuster is sufficient, the apertures might be defined at the end of curved or otherwise non-orthogonal channels or cavities through the adjuster thickness, so that the entrance to the aperture on the lower side of the adjuster 25 (facing the lower portion 24) has a different position to the aperture exit on the upper side (facing the tank base 20). In such a configuration, the lower openings 34 in the lower portion 24 need not be aligned with the upper openings 38 in the tank base 20, but will still be connected by the aperture channels in the adjuster.

An adjuster in an upstream location is not applicable only to the side-by-side "box" type of the vapour provision system such as the Figure 1 example. It can also be utilised in a linear configuration in which the battery section is axially displaced from the aerosol delivery section (instead of transversely displaced) to give an elongate device.

Figure 8 shows a schematic side external view of an elongate vapour provision system 100 provided with an adjuster in accordance with embodiments on the invention. The system 100 comprises an aerosol delivery section 14 having a mouthpiece 22 at one end. Connected to its other end via a mechanical and electrical connection is a battery section 12. An air inlet 29 is provided towards the end of the aerosol delivery section 14 to intake air A for flow along an airflow path inside the aerosol delivery section 14 to the mouthpiece 22. Downstream of the air inlet 29, with respect to this airflow direction, is located an airflow adjuster 25, having a ridged outer surface to facilitate gripping by a user to rotate the adjuster for variable airflow in the airflow path. Principles and features of airflow adjusters discussed above with regard to side-by-side devices are applicable also to elongate devices such as this example.

Other configurations of airflow adjuster for providing variation of airflow at a location downstream from the air inlet are also contemplated.

Figure 9 shows a first example of an alternative configuration, illustrated as a cross-sectional view looking down onto a lower portion 124 of an aerosol delivery portion which has (or has a connection to) an air inlet (not shown), such as the lower portion 24 of Figure 2. The air inlet is connected via a channel (not shown) to a lower opening 134 in the upper face 136 of the lower portion 124. This defines a first portion of the airflow path, and will connect with a second portion defined through a tank portion which in use is connected to the lower portion 124 (as in Figure 2). In this example, the airflow adjuster has the form of shutter 101. The shutter 101 is sector-shaped (other shapes are not precluded), and pivotally attached to the upper face 136 at a pivot 102 so that it can slide over the upper face 136 about the pivot 102. Sliding of the shutter 101 in this manner allows the shutter 101 to be moved over the opening 134 by a variable amount, thereby more or less restricting the airflow path and varying the amount of air that can be drawn through the path. Abutments 103 or other movement limiters (such as a recess within which the shutter slides) can be provided to prevent total blockage of the opening 134 by the shutter 101. A handle portion 104 can be provided on the edge of the shutter 101 remote from the pivot 102 so as to protrude beyond the outer wall of the aerosol delivery section, by which a user can manipulate the shutter between the maximum and minimum airflow positions. Any shape of shutter and size, shape and position of opening can be used. For example, the opening can be centrally located along the axis X (see Figure 2), or off-set as in Figure 9. The shutter could instead by pivotally mounted to the underside face of the tank base to slide over the upper opening.

Figure 10 shows a second example of an alternative configuration. The view is a plan view of the adjuster, which in this example is formed from a plurality of overlapping shutters 201 arranged in the manner of a mechanical camera shutter. The shutters can be extended and retracted to vary the size of a central aperture 242, which is arranged over an opening 234 in a lower portion or a tank base. Thus, the effective size of the opening 234 is varied as the shutters move, since it is limited to the size of the central aperture 242. A control 204 is provided on the outer surface of the aerosol delivery portion for a user to open and close the shutters 201. Appropriate sizing of the shutters can allow for a permanent central aperture defined by a minimum possible aperture (i.e. the shutters cannot completely cover the opening 234) so that the airflow path cannot be fully blocked.

Other configurations of airflow adjuster will be evident to the skilled person that are also compatible with an arrangement in which the adjuster is located downstream from air inlet. Any arrangement which allows the bore of the airflow passage to be variably increased and decreased by a user at a downstream position might be utilised.

In any embodiment, the airflow adjuster may be configured to allow continuous adjustment between the position corresponding to maximum airflow (maximum bore of the airflow path) and the position corresponding to minimum airflow (minimum bore of the airflow path), or to allow adjustment between two or more predetermined positions only.

While the invention has been described with regard to example devices and components which are substantially circular in cross-section, it is not so limited, and is applicable to aerosol and vapour provision devices and systems of other shapes.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims and that other embodiments may be utilised and modifications may be made without departing from the scope of the claimed invention.

## Claims

1. An aerosol delivery component (14) for a vapour provision system (10), comprising:
an air inlet (29);
an airflow path (32, 40) connected to the air inlet and extending through the aerosol delivery component;
a heating element in the airflow path for vaporising a source liquid delivered from a tank (16) to form an aerosol;
and
an airflow adjuster (25) for varying a level of airflow along the airflow path, the airflow adjuster allowing a user to alter an amount of air able to flow along the airflow path, and located in the airflow path downstream from the air inlet and upstream of the heating element;
wherein the aerosol delivery component is mechanically and electrically connectable to a battery section (12) of the vapour provision system, the battery section housing a battery (26) connectable to the aerosol delivery component to provide electrical power to the heating element in the aerosol delivery component and said battery section having one or more buttons or switches (30) operable by the user to deliver electrical power to one or more components in the aerosol delivery component.

2. An aerosol delivery component according to claim 1, in which the airflow adjuster is spaced from the air inlet by a portion (32) of the airflow path.

3. An aerosol delivery component according to claim 1 or claim 2, in which the airflow adjuster comprises an element (25; 101) movable into and out of the airflow path to alter a size of a bore of the airflow path at the location of the element.

4. An aerosol delivery component according to claim 3, in which the element is movable by rotation or sliding.

5. An aerosol delivery component according to claim 4, in which the aerosol delivery component comprises a first section (24) having a first portion (32) of the airflow path defined therein and a second section (20) having a second portion (40) of the airflow path defined therein, and the airflow adjuster comprises a planar element (25) interposed between the first section and the second section and having at least one aperture (42) through the planar element, the planar element being rotatable such that the aperture can be brought into and out of alignment with the airflow path.

6. An aerosol delivery component according to claim 5, and further comprising a rotation limiter configured to prevent rotation of the planar element into a position in which there is no alignment of the aperture with the airflow path.

7. An aerosol delivery component according to claim 5, in which the at least one aperture comprises at least two apertures (42) circumferentially spaced around the planar element and spaced apart by a circumferential distance (S) which is less than a circumferential dimension (D) of the airflow path, so that there is at least partial alignment of an aperture and the airflow path for all rotational positions of the planar element.

8. An aerosol delivery component according to any one of claims 5 to 7, in which one or both of the at least one aperture and the airflow path at the location of the planar element has a dimension in a radial direction with respect to the axis of rotation (X) of the planar element that is not constant over the circumferential extent of the aperture or path.

9. An aerosol delivery component according to claim 8, in which the said dimension in the radial direction increases over the circumferential extent.

10. An aerosol delivery component according any one of claims 1 to 9, in which the airflow path comprises two or more airflow paths at the location of the adjuster.

11. A vapour provision system (10) comprising an aerosol delivery component (14) according to any one of claims 1 to 10, and a battery section (12) for housing a battery (26) connectable to the aerosol delivery component to provide electrical power to the heating element in the aerosol delivery component.

## Patentansprüche

1. Aerosolabgabekomponente (14) für ein Dampfversorgungssystem (10), umfassend:
einen Lufteinlass (29);
einen Luftströmungsweg (32, 40), der mit dem Lufteinlass verbunden ist und sich durch die Aerosolabgabekomponente erstreckt;
ein Heizelement im Luftströmungsweg zum Verdampfen einer aus einem Tank (16) abgegebenen Ausgangsflüssigkeit, um ein Aerosol zu bilden;
und
einen Luftstromregler (25) zum Variieren einer Luftstromstärke entlang des Luftströmungswegs, wobei der Luftstromregler es einem Benutzer ermöglicht, eine entlang des Luftströmungswegs strömende Luftmenge zu verändern, und sich im Luftströmungsweg stromabwärts des Lufteinlasses und stromaufwärts des Heizelements befindet;
wobei die Aerosolabgabekomponente mechanisch und elektrisch mit einem Batterieabschnitt (12) des Dampfversorgungssystems verbindbar ist, wobei der Batterieabschnitt eine Batterie (26) enthält, die mit der Aerosolabgabekomponente verbindbar ist, um das Heizelement in der Aerosolabgabekomponente mit elektrischer Leistung zu versorgen, und wobei der Batterieabschnitt eine oder mehrere Tasten oder Schalter (30) aufweist, die vom Benutzer betätigt werden können, um eine oder mehrere Komponenten in der Aerosolabgabekomponente mit elektrischer Leistung zu versorgen.

2. Aerosolabgabekomponente nach Anspruch 1, wobei der Luftstromregler zum Lufteinlass durch einen Abschnitt (32) des Luftströmungswegs beabstandet ist.

3. Aerosolabgabekomponente nach Anspruch 1 oder Anspruch 2, wobei der Luftstromregler ein Element (25; 101) umfasst, das in den Luftströmungsweg hinein und aus diesem heraus bewegbar ist, um eine Größe einer Bohrung des Luftströmungswegs an der Stelle des Elements zu verändern.

4. Aerosolabgabekomponente nach Anspruch 3, wobei das Element durch Drehung oder Verschieben bewegbar ist.

5. Aerosolabgabekomponente nach Anspruch 4, wobei die Aerosolabgabekomponente ein erstes Teilstück (24) mit einem darin definierten ersten Abschnitt (32) des Luftströmungswegs und ein zweites Teilstück (20) mit einem darin definierten zweiten Abschnitt (40) des Luftströmungswegs umfasst und der Luftstromregler ein ebenes Element (25) umfasst, das zwischen dem ersten Teilstück und dem zweiten Teilstück angeordnet ist und mindestens eine Öffnung (42) durch das ebene Element aufweist, wobei das ebene Element so drehbar ist, dass die Öffnung in und außer Ausrichtung mit dem Luftströmungsweg bringbar ist.

6. Aerosolabgabekomponente nach Anspruch 5, ferner umfassend einen Drehbegrenzer, der so konfiguriert ist, dass er eine Drehung des ebenen Elements in eine Position verhindert, in der keine Ausrichtung der Öffnung mit dem Luftströmungsweg vorliegt.

7. Aerosolabgabekomponente nach Anspruch 5, wobei die mindestens eine Öffnung mindestens zwei Öffnungen (42) umfasst, die in Umfangsrichtung entlang des ebenen Elements beabstandet sind und einen Umfangsabstand (S) zueinander aufweisen, der kleiner ist als eine Umfangsabmessung (D) des Luftströmungswegs, so dass für alle Drehpositionen des ebenen Elements zumindest eine teilweise Ausrichtung einer Öffnung und des Luftströmungswegs vorliegt.

8. Aerosolabgabekomponente nach einem der Ansprüche 5 bis 7, wobei die mindestens eine Öffnung und/oder der Luftströmungsweg an der Stelle des ebenen Elements eine Abmessung in radialer Richtung in Bezug auf die Drehachse (X) des ebenen Elements aufweisen, die über die Umfangserstreckung der Öffnung oder des Wegs nicht konstant ist.

9. Aerosolabgabekomponente nach Anspruch 8, wobei die Abmessung in radialer Richtung über die Umfangserstreckung zunimmt.

10. Aerosolabgabekomponente nach einem der Ansprüche 1 bis 9, wobei der Luftströmungsweg an der Stelle des Reglers zwei oder mehr Luftströmungswege umfasst.

11. Dampfversorgungssystem (10), das eine Aerosolabgabekomponente (14) nach einem der Ansprüche 1 bis 10 sowie einen Batterieabschnitt (12) zur Aufnahme einer Batterie (26) umfasst, die mit der Aerosolabgabekomponente verbindbar ist, um das Heizelement in der Aerosolabgabekomponente mit elektrischer Leistung zu versorgen.

## Revendications

1. Composant de distribution d'aérosol (14) pour un système de fourniture de vapeur (10), comprenant :
une entrée d'air (29) ;
un trajet d'écoulement d'air (32, 40) relié à l'entrée d'air et s'étendant à travers le composant de distribution d'aérosol ;
un élément chauffant dans le trajet d'écoulement d'air pour vaporiser un liquide source distribué à partir d'un réservoir (16) pour former un aérosol ;
et
un dispositif de réglage d'écoulement d'air (25) pour faire varier un niveau d'écoulement d'air le long du trajet d'écoulement d'air, le dispositif de réglage d'écoulement d'air permettant à un utilisateur de modifier une quantité d'air pouvant s'écouler le long du trajet d'écoulement d'air, et situé dans le trajet d'écoulement d'air en aval de l'entrée d'air et en amont de l'élément chauffant ;
dans lequel le composant de distribution d'aérosol peut être relié mécaniquement et électriquement à une section de batterie (12) du système de fourniture de vapeur, la section de batterie logeant une batterie (26) pouvant être reliée au composant de distribution d'aérosol pour fournir de l'énergie électrique à l'élément chauffant dans le composant de distribution d'aérosol et ladite section de batterie ayant un ou plusieurs boutons ou commutateurs (30) actionnables par l'utilisateur pour fournir de l'énergie électrique à un ou plusieurs composants dans le composant de distribution d'aérosol.

2. Composant de distribution d'aérosol selon la revendication 1, dans lequel le dispositif de réglage d'écoulement d'air est espacé de l'entrée d'air par une partie (32) du trajet d'écoulement d'air.

3. Composant de distribution d'aérosol selon la revendication 1 ou la revendication 2, dans lequel le dispositif de réglage d'écoulement d'air comprend un élément (25 ; 101) mobile dans et hors du trajet d'écoulement d'air pour modifier une taille d'un alésage du trajet d'écoulement d'air au niveau de l'emplacement de l'élément.

4. Composant de distribution d'aérosol selon la revendication 3, dans lequel l'élément est mobile par rotation ou coulissement.

5. Composant de distribution d'aérosol selon la revendication 4, dans lequel le composant de distribution d'aérosol comprend une première section (24) ayant une première partie (32) du trajet d'écoulement d'air définie dans celle-ci et une seconde section (20) ayant une seconde partie (40) du trajet d'écoulement d'air définie dans celle-ci, et le dispositif de réglage d'écoulement d'air comprend un élément plan (25) interposé entre la première section et la seconde section et ayant au moins une ouverture (42) à travers l'élément plan, l'élément plan pouvant tourner de telle sorte que l'ouverture peut être amenée en alignement et hors d'alignement avec le trajet d'écoulement d'air.

6. Composant de distribution d'aérosol selon la revendication 5, et comprenant en outre un limiteur de rotation configuré pour empêcher la rotation de l'élément plan dans une position dans laquelle il n'y a pas d'alignement de l'ouverture avec le trajet d'écoulement d'air.

7. Composant de distribution d'aérosol selon la revendication 5, dans lequel l'au moins une ouverture comprend au moins deux ouvertures (42) espacées circonférentiellement autour de l'élément plan et espacées d'une distance circonférentielle (S) qui est inférieure à une dimension circonférentielle (D) du trajet d'écoulement d'air, de sorte qu'il y a au moins un alignement partiel d'une ouverture et du trajet d'écoulement d'air pour toutes les positions de rotation de l'élément plan.

8. Composant de distribution d'aérosol selon l'une quelconque des revendications 5 à 7, dans lequel l'un ou les deux de l'au moins une ouverture et du trajet d'écoulement d'air au niveau de l'emplacement de l'élément plan a une dimension dans une direction radiale par rapport à l'axe de rotation (X) de l'élément plan qui n'est pas constante sur l'étendue circonférentielle de l'ouverture ou du trajet.

9. Composant de distribution d'aérosol selon la revendication 8, dans lequel ladite dimension dans la direction radiale augmente sur l'étendue circonférentielle.

10. Composant de distribution d'aérosol selon l'une quelconque des revendications 1 à 9, dans lequel le trajet d'écoulement d'air comprend deux ou plusieurs trajets d'écoulement d'air au niveau de l'emplacement du dispositif de réglage.

11. Système de fourniture de vapeur (10) comprenant un composant de distribution d'aérosol (14) selon l'une quelconque des revendications 1 à 10, et une section de batterie (12) pour loger une batterie (26) pouvant être reliée au composant de distribution d'aérosol pour fournir de l'énergie électrique à l'élément chauffant dans le composant de distribution d'aérosol.
